(19) Europäisches Patentamt
European Patent Office
Office européen des brevets

(11) Publication number **0 023 358**
**A1**

# (12) EUROPEAN PATENT APPLICATION

(21) Application number: **80104445.4**

(22) Date of filing: **28.07.80**

(51) Int. Cl.³: **C 07 D 237/24**

(30) Priority: **30.07.79 US 61589**

(43) Date of publication of application: **04.02.81 Bulletin 81/5**

(84) Designated Contracting States: **AT BE CH DE FR GB IT LI NL SE**

(71) Applicant: **Rohm and Haas Company**
**Independence Mall West**
**Philadelphia, Pennsylvania 19105(US)**

(72) Inventor: **Carlson, Glenn Richard**
**305 Britt Road**
**N. Wales, Pa.(US)**

(74) Representative: **Deufel, Paul et al,**
**Patentanwälte MÜLLER-BORé-DEUFEL SCHÖN-HERTEL**
**Siebertstrasse 4**
**D-8000 München 86(DE)**

## (54) Process for the preparation of pyridazine derivatives.

(57) A 1-aryl-1, 4-dihydro-4-oxopyridazine carboxamide of the formula:

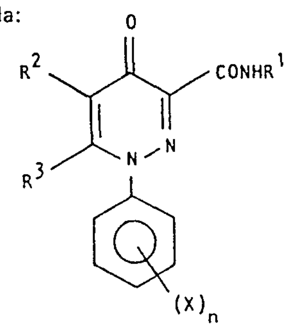

(1)

is prepared by an improved process which comprises reacting, in a liquid medium, ammonia or an amine of the formula $R^1NH_2$ with a hydrazone of the formula:

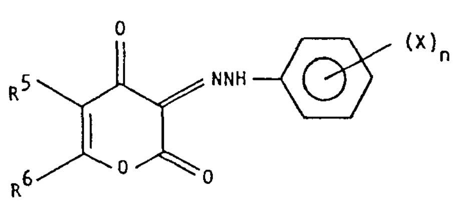

In the above formulae;

$R^1$ is hydrogen, $(C_1-C_6)$alkyl, $(C_1-C_4)$alkoxy, $(C_1-C_4)$alkyl, $(C_3-C_8)$cycloalkyl, $(C_3-C_8)$cycloalkyl $(C_1-C_4)$alkyl, halo$(C_1-C_6)$alkyl, $(C_1-C_4)$ alkoxycarbonyl$(C_1-C_4)$alkyl, benzyl or benzyl the ring of which is substituted with up to two of the same or different substituents selected from chloro, bromo, methyl, ethyl, methoxy, cyano, nitro and trifluoromethyl;

$R^2$ and $R^3$ are hydrogen or $(C_1-C_4)$alkyl;

X is selected from halo, trihalomethyl, nitro, $(C_1-C_4)$-alkyl, $(C_1-C_4)$alkoxy, $(C_1-C_4)$alkylthio, $(C_1-C_4)$alkylsulfinyl, $(C_1-C_4)$alkylsulfonyl, cyano and carboxy, the X substituents being the same or different when n is > 1; and n is 0 or an integer from 1-3

The amides of Formula I find use as plant growth regulators.

EP 0 023 358 A1

PATENTANWÄLTE
MÜLLER-BORÉ · DEUFEL
SCHÖN · HERTEL
8 MÜNCHEN 86 · SIEBERTSTR. 4
TEL (089) 47 40 05 · TELEX 5-24 85



# PROCESS FOR THE PREPARATION OF PYRIDAZINE DERIVATIVES

This invention concerns an improved process for the preparation of pyridazine derivatives, namely 1-aryl-1,4-dihydro-4-oxopyridazine-3-carboxamides.

The preparation of 1-aryl-1,4-dihydro-4-oxo-pyridazine-3-carboxylic acids via the rearrangement of a hydrazone in hot aqueous mineral acids or hot aqueous base is disclosed in Belgian Patent 864,704. These methods are additionally disclosed in British Patent Specification 762,141; in United States Patent 2,835,671, in Helvetica Chimica Acta, 39, 1741 (1956), in the Journal of the American Chemical Society (JACS), 78, 624 (1955) and in JACS, 70, 2253 (1948).

That 1-aryl-1,4-dihydro-4-oxopyridazine carboxylic acids can be esterified via Fischer esterification is disclosed in Helvetica Chimica Acta 39, 1741 (1956) and United States Patent 2,835,671. The reaction of these esters with ammonia or primary alkyl amines to form 1-aryl-1,4-dihydro-4-oxopyridazine-3-carboxamides is also disclosed in this reference. The Fischer esterification method requires the isolation of the carboxylic acid prior to the esterification reaction. Fischer esterification reactions generally produce a crude product that is highly colored and rather impure. Several recrystallizations are often required to obtain material of adequate purity for analysis and testing. Fischer esterification generally requires several hours to several days before completion and gives low yields. Moreover, amidation requires additional time and subsequent lowering of yields.

In accordance with the invention, there is provided a process for the preparation of a 1-aryl-1, 4-dihydro-4-oxopyridazine carboxamide of the formula:

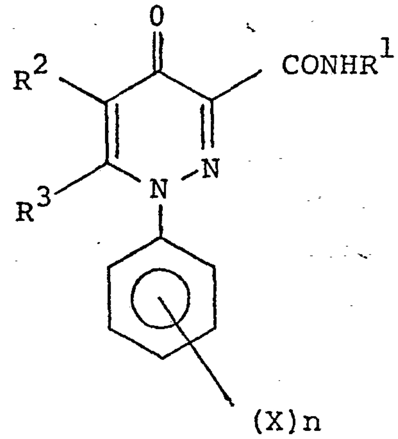

(I)

which comprises reacting, in a liquid medium, ammonia or an amine of the formula $R^1NH_2$ (II) with a hydrazone of the formula:

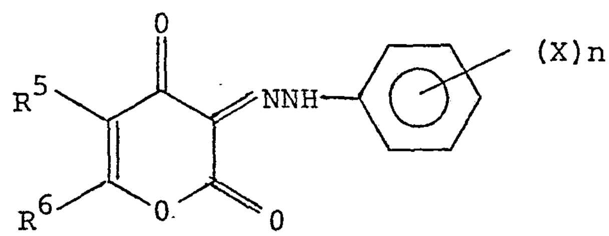

(III)

where, in the above formulae,

$R^1$ is hydrogen, $(C_1-C_6)$alkyl, $(C_1-C_4)$alkoxy-$(C_1-C_4)$alkyl, $(C_3-C_8)$cycloalkyl, $(C_3-C_8)$cycloalkyl$(C_1-C_4)$alkyl, halo$(C_1-C_6)$alkyl, $(C_1-C_4)$alkoxy-carbonyl$(C_1-C_4)$alkyl, benzyl or benzyl the ring of which is substituted with up to two of the same or different substituents selected from chloro, bromo, methyl, ethyl, methoxy, cyano, nitro and trifluoromethyl;

$R^2$ and $R^3$ are hydrogen or $(C_1-C_4)$alkyl;

X is selected from halo, trihalomethyl, nitro, $(C_1-C_4)$alkyl, $(C_1-C_4)$alkoxy, $(C_1-C_4)$alkylthio, $(C_1-C_4)$alkylsulfinyl, $(C_1-C_4)$alkylsulfonyl, cyano and carboxy, the X substituents being the same or different when n is $>1$; and

n is zero or an integer from one to three.

The amides of Formula I are useful as plant growth regulants and in particular as chemical hybridization agents.

The reaction between the hydrazone and ammonia or amine is generally conducted at a temperature from 25°C to 150°C and preferably 40°C to 80°C and is also generally conducted in the presence of an inert solvent, particularly a cosolvent system. Also there will usually be used at least one mole of $R^1NH_2$ reactant per mole of hydrazone. Furthermore it is convenient that the solvent medium contains excess $R^1NH_2$ reactant as a solvent.

The term "alkyl" as used in this specification is meant to include both branched and straight chain alkyl groups; also by the use of an unqualified term such as butyl, it is meant to include all the possible isomers. The term "halo" used herein means fluoro, chloro, bromo or iodo.

Typical compounds of Formula (II) that can be utilized in the process of the invention include ammonia, methylamine, ethylamine, isopropylamine, _n_-propylamine, _n_-butylamine, _sec_-butylamine, _tert_-butylamine, _n_-pentylamine, _n_-hexylamine, methoxymethylamine, cyclohexylamine, cyclohexylmethylamine, benzylamine, chlorobenzylamine, glycine methyl ester and glycine.

Typical inert solvents that can be utilized in

cosolvent systems include aromatic hydrocarbons such as benzene, toluene, xylene and lower aliphatic alcohols such as methanol, ethanol, _n_-propanol, _n_-butanol and water.

A preferred process of this invention comprises using a hydrazone of Formula (III) wherein $R^2$ and $R^3$ are hydrogen, methyl or ethyl (particularly hydrogen or methyl); X is fluoro, chloro, bromo, iodo, trifluoromethyl, nitro, methyl, methoxy, methylthio, methylsulfonyl or methylsulfinyl (particularly chloro, bromo or iodo) and n is zero or the integer one or two. In this preferred process, there is also used ammonia or an amine of Formula (II) wherein $R^1$ is methyl, ethyl, propyl, butyl, cyclohexyl, methoxycarbonylmethyl or benzyl. Finally, in this preferred process, there is used an excess of the Formula (II) compound as reaction solvent and there is used a temperature from $40^oC$ to $80^oC$.

Another feature of this invention involves the addional step of cooling the resultant reaction mixture, adding it in water, collecting the desired product by filtration or extracting it with an appropriate solvent. Appropriate solvents which can be utilized in this extraction procedure include methylene chloride, chloroform, carbon tetrachloride, benzene, toluene, xylene and diethylether.

The isolated amide can, if desired, be treated with an aqueous or alcoholic solution of an alkali metal hydroxide such as sodium hydroxide or potassium hydroxide at reflux to form the corresponding sodium or potassium salt of the desired product. Alternatively, the amide can be treated at reflux with concentrated hydrochloric acid or 20% to 30% sulfuric acid until the free carboxylic acid is obtained.

The following examples are presented to

illustrate the process of the present invention. In the examples, percentages are on a weight basis unless otherwise indicated and degrees are in degrees Celsius.

EXAMPLE 1

Preparation of N-ethyl-1-phenyl-1,4-dihydro-4-oxo-6-methyl-3-carboxamide

A suspension of 10 g of 3-phenylhydrazono-4-oxo-6-methyl-2-pyrone (0.0436 moles) in 100 ml of 35% aqueous ethylamine was refluxed for ten minutes, then cooled in an ice-bath. The crystals that formed were collected by filtration and recrystallized from methylene chloride/ether to yield 6.8 g of the desired product (61% yield), mp = 169-70°C.

EXAMPLE 2

Preparation of N-ethyl 1-(4-chlorophenyl)-1,4-dihydro-4-oxo-6-methyl-3-carboxamide

A suspension of 8.0 g of 3-(4-chlorophenyl-hydrazono)-4-oxo-6-methyl-2-pyrone (0.0303 moles) in 100 ml of 35% aqueous ethylamine was very briefly refluxed. As soon as the hydrazone dissolved the mixture was cooled and diluted with water. The solid that formed was collected by filtration and recrystallized from methylene chloride/ether to yield 2.0 g of the desired amide (23% yield), mp = 154-6°C.

EXAMPLE 3

Preparation of N-methyl 1-(4-chlorophenyl)-1,4-dihydro-4-oxo-6-methylpyridazine-3-carboxamide

A suspension of 8.0 g of 3-(4-chlorophenyl-hydrazono)-4-oxo-6-methyl-2-pyrone (0.0303 moles) in 100 ml of 40% aqueous methylamine was refluxed for 15 minutes. The resulting clear orange solution was cooled to 10°C. The precipitate that formed was collected by filtration and recrystallized from methylene chloride/ether to yield 5.6 g of the desired amide (66%), mp = 237-8°C.

EXAMPLE 4

Preparation of 1-(4-chlorophenyl)-1,4-dihydro-4-oxo-6-methyl-pyridazine-3-carboxamide

A suspension of 9.0 g of 3-(4-chlorophenylhydrazono)-4-oxo-6-methyl-2-pyrone (0.0341 moles), 90 ml of concentrated ammonium hydroxide and 60 ml of methanol was refluxed for thirty minutes. The mixture was then cooled to 10°C. The solid that precipitates under these conditions was filtered and recrystallized from methylene chloride/ether to yield 6.6 g of the desired amide (73%), mp = 226-7°C.

EXAMPLE 5

Preparation of N-propyl-1-(4-chlorophenyl)-1,4-dihydro-4-oxo-6-methylpyridazine-3-carboxamide

A suspension of 8.0 g of 3-(4-chlorophenylhydrazono)-4-oxo-6-methyl-2-pyrone (0.0303 moles) in 25 g *n*-propylamine (0.42 moles) and 75 ml of water was refluxed very briefly, then cooled immediately. Extraction with methylene chloride (2 x 100 ml) yielded a brown oil after evaporation. Recrystallization of this material from methylene chloride/ether yielded 6.2 g of the desired amide (67% yield), mp = 134-5°C.

EXAMPLE 6

Preparation of N-hexyl-1-(4-chlorophenyl)-1,4-dihydro-4-oxo-6-methyl-pyridazine-3-carboxamide

A suspension of 10 g of 3-(4-chlorophenylhydrazono)-4-oxo-6-methyl-2-pyrone (0.0379 moles), 12 g of *n*-hexylamine (0.12 moles), 100 ml of methanol and 50 ml of water was refluxed for five minutes. The resulting deep orange solution was cooled and diluted with 300 ml of water. This mixture was then extracted with methylene chloride (3 x 100 ml). Evaporation of the organic extracts yielded an orange oil that crystallized on standing. Recrystallization from

methylene chloride/ether/hexane yielded 4.5 g of the desired amide (34%), mp = 113-8°C.

EXAMPLE 7

Preparation of N-methoxycarbonylmethyl-1-(4-chlorophenyl)-1,4-dihydro-4-oxo-6-methylpyridazine-3-carboxamide

A suspension of 11.4 g of 3-(4-chlorophenylhydrazono)-4-oxo-6-methyl-2-pyrone (0.0435 moles) in 100 ml of methanol containing 16.3 g of glycine methyl ester hydrochloride (0.130 moles) was slowly added to 10.4 g of 50% aqueous sodium hydroxide. The resulting mixture was refluxed for 15 minutes, cooled, and poured into 400 ml of water. Extraction with 2 x 150 ml methylene chloride yielded a yellow solid. Recrystallization of this material from toluene yielded 6.3 g of the desired amide (43% yield), mp = 166-8°C.

As stated above the improved process of the present invention produces higher yields of purer product at much shorter reaction times than processes utilizing Fischer esterification followed by amidation. Table I presented below gives a comparison of the process of the present invention as compared to standard Fischer esterification followed by amidation.

TABLE I

Present Process Versus Fischer Esterification/ Amidation Process

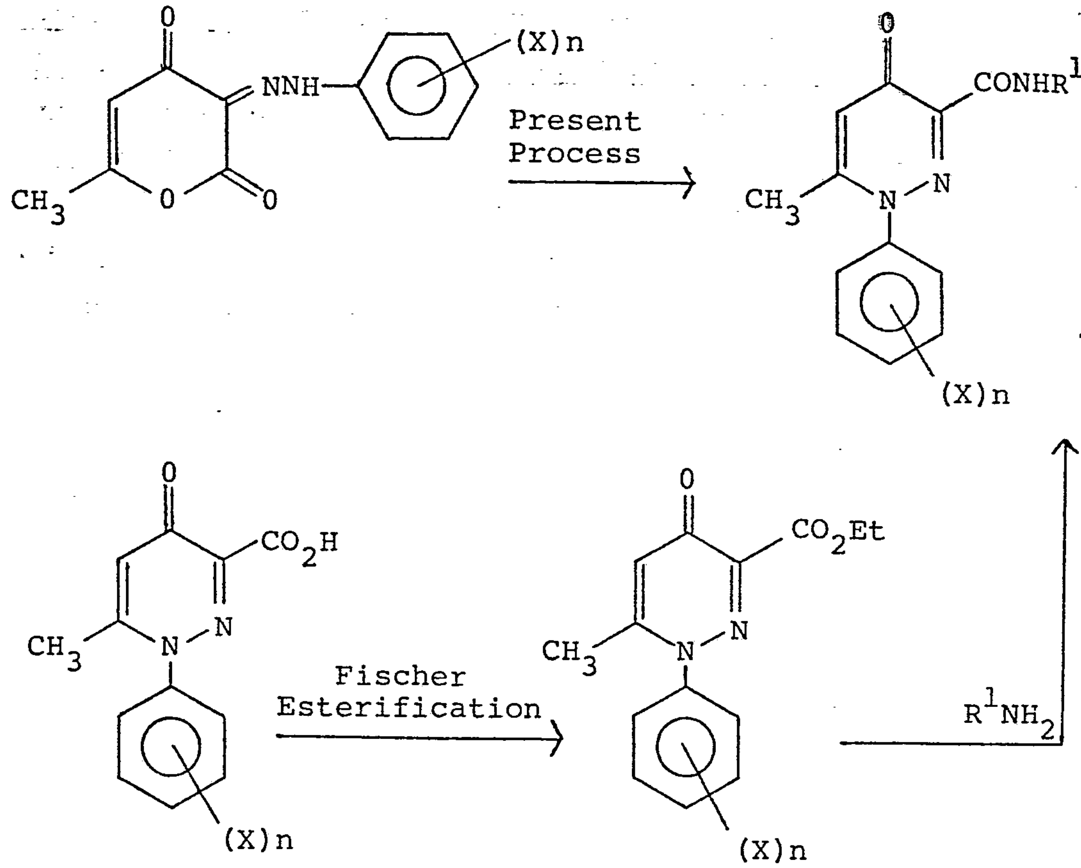


| $R^1$ | (X) n | Fischer Esterification/ Amidation Process Yield | Combined Reaction Time | Present Process Yield | Present Process Reaction Time |
|---|---|---|---|---|---|
| H | 4-Cl | $60^{a)}$x76=46 | 72+2=74 hrs. | 73 | 30 minutes |
| Et | H | $35^{a)}$x77=25 | 20+2.5=22.5 hrs | 61 | 10 minutes |

a) Yield of corresponding ethyl ester.

Claims:

1. A process for the preparation of a 1-aryl-1, 4-dihydro-4-oxopyridazine carboxamide of the formula:

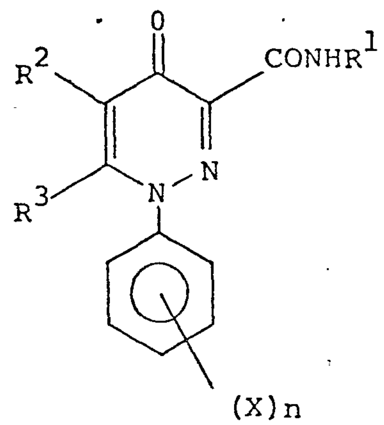

(I)

which comprises reacting, in a liquid medium, ammonia or an amine of the formula $R^1NH_2$ with a hydrazone of the formula:

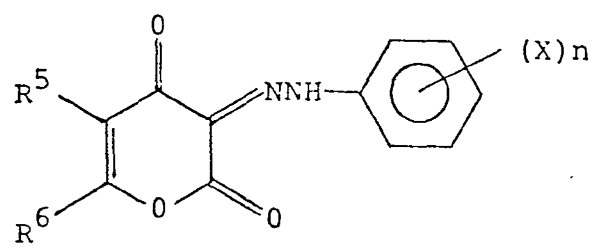

where, in the above formulae,

$R^1$ is hydrogen, $(C_1-C_6)$alkyl, $(C_1-C_4)$alkoxy, $(C_1-C_4)$alkyl, $(C_3-C_8)$cycloalkyl, $(C_3-C_8)$cycloalkyl$(C_1-C_4)$alkyl, halo$(C_1-C_6)$alkyl, $(C_1-C_4)$alkoxycarbonyl-$(C_1-C_4)$alkyl, benzyl or benzyl the ring of which is substituted with up to two of the same or different substituents selected from chloro, bromo, methyl, ethyl, methoxy, cyano, nitro and trifluoromethyl;

BAD ORIGINAL

$R^2$ and $R^3$ are hydrogen or $(C_1-C_4)$alkyl;
X is selected from halo, trihalomethyl, nitro, $(C_1-C_4)$alkyl, $(C_1-C_4)$alkoxy, $(C_1-C_4)$alkylthio, $(C_1-C_4)$alkylsulfinyl, $(C_1-C_4)$alkylsulfonyl, cyano and carboxy, the X substituents being the same or different when n is $>1$; and n is 0 or an integer from 1-3.

2. A process as claimed in Claim 1, wherein there is used at least one mole of the $R^1NH_2$ reactant per mole of hydrazone and the reaction is carried out in solution at a temperature from 25°C to 150°C.

3. A process as claimed in Claim 2, wherein the reaction is carried out at a temperature from 40°C to 80°C.

4. A process as claimed in Claim 2 or 3, wherein the solvent medium contains excess $R^1NH_2$ reactant as a solvent.

5. A process as claimed in any one of Claims 2-4, wherein $R^1$ is hydrogen, methyl, ethyl, propyl, butyl, hexyl, methoxycarbonylmethyl, cyclohexyl or benzyl, $R^2$ and $R^3$ are hydrogen, methyl or ethyl, and X is fluoro, chloro, bromo, iodo, trifluoromethyl, nitro, methyl, methoxy, methylthio, methylsulfinyl or methylsulfonyl.

6. A process as claimed in Claim 5, wherein $R^1$ is hydrogen, methyl, ethyl, propyl or butyl. $R^2$ and $R^3$ are hydrogen or methyl and X is chloro, bromo or iodo.

7. A process according to any one of Claims 3-6 which comprises the additional step of cooling the resultant reaction mixture, adding it to water and

extracting the desired product with a solvent.

8. A process as claimed in any one of Claims 1-7 as applied to the preparation of 1-(4-chlorophenyl)-1, 4-dihydro-4-oxypyridazine-3-carboxamide, N-hexyl-1-(4-chlorophenyl)-1, 4-dihydro-4-oxopyridazine-3-carboxamide, N-methoxycarbonylmethyl-1-(4-chlorophenyl)-1,4-dihydro-4-oxo-6-methylpyridazine-3-carboxamide or 1-phenyl-1,4-dihydro-4-oxopyridazine-3-carboxamide.

9. A process as claimed in any one of Claims 1-7, wherein the hydrazone reactant is 3-(4-chlorophenylhydrazone)-4-oxo-6-methyl-2-pyrone.

0023358

European Patent Office

# EUROPEAN SEARCH REPORT

Application number EP 80 10 4445

| Category | Citation of document with indication where appropriate, of relevant passages | Relevant to claim | CLASSIFICATION OF THE APPLICATION (Int Cl.³) |
|---|---|---|---|
| | THE CHEMISTRY OF HETEROCYCLIC COMPOUNDS, Edit. A. Weissberger and E.C. Taylor, A series of monographs, John Wiley & Sons, New York, US. An interscience publication, 1973, "Pyridazines", Ed. by R.N. Castle. * Page 408 * | 1 | C 07 D 237/24 |
| | -- | | |
| | FR - A - 2 383 605 (ROHM & HAAS) * Pages 1,6,7,8 * | 1 | TECHNICAL FIELDS SEARCHED (Int Cl.³) |
| | -- | | |
| | DE - A - 1 095 287 (LE PETIT) * Claim 3, lines 15-32 * | 1 | C 07 D 237/24 |
| | ---- | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 07-11-1980 | VERHULST |

EPO Form 1503.1 06.78